# EUROPEAN PATENT SPECIFICATION

(11) **EP 1 992 290 B1**
(45) Date of publication and mention of the grant of the patent: **26.10.2011**
(21) Application number: 07706311.3
(22) Date of filing: 07.02.2007
(51) Int. Cl.: A61B 8/12, H04R 1/40, H04R 19/00, H04R 31/00

(54) **ULTRASONIC VIBRATOR AND BODY CAVITY ULTRASONOGRAPH HAVING THE ULTRASONIC VIBRATOR**
ULTRASCHALLVIBRATOR UND KÖRPERHÖHLEN-ULTRASCHALLGERÄT MIT ULTRASCHALLVIBRATOR
VIBRATEUR A ULTRASONS ET ULTRASONOGRAPHE DE CAVITE DE CARTE POSSEDANT LE VIBRATEUR A ULTRASONS

(30) Priority: 03.03.2006 JP 2006057121
(43) Date of publication of application: 19.11.2008
(73) Proprietor: Olympus Medical Systems Corp., Tokyo 151-0072 (JP)
(72) Inventor: ADACHI, Hideo, Tokyo 1510072 (JP); WAKABAYASHI, Katsuhiro, Tokyo 1510072 (JP); MIZUNUMA, Akiko, Tokyo 1510072 (JP); SAWADA, Yukihiko, Tokyo 1510072 (JP); IMAHASHI, Takuya, Tokyo 1510072 (JP); FUJIMURA, Takanao, Tokyo 1510072 (JP); DOH, Ki, Tokyo 1510072 (JP)
(74) Representative: von Hellfeld, Axel
(86) International application number: PCT/JP2007/000063
(87) International publication number: WO 2007/099696

(56) References cited:
- JP-A- 2004 350 703
- JP-A- 2004 350 704
- YONGRAE R. ET AL.: 'Finite element modeling of capacitor micromachined ultrasonic transducers' PROC. IEEE ULTRASON. SYMP. vol. 1, October 2000, pages 905 - 908, XP010541732

## Description

### Technical Field

The present invention relates to a MUT (Micromachined Ultrasound Transducer).

### Background Art

A commonly employed ultrasound diagnosis method is one in which ultrasound is transmitted to the walls of a body cavity and the state of the body cavity is converted into images on the basis of the echo signals thereof for the purpose of making diagnoses. An ultrasound endoscope is one of the devices used for this ultrasound diagnosis method.

The ultrasound endoscope has an ultrasound probe at the distal end of an insertion tube to be inserted into body cavities, and this ultrasound probe converts electric signals into ultrasound in order to transmit the ultrasound to body cavities and also receives ultrasound reflected in the body cavities in order to convert the received ultrasound into electric signals.

Conventionally, ceramic piezoelectric material PZT (lead zirconate titanate) has been used for piezoelectric elements that convert electric signals into ultrasound. However, capacitive micromachined ultrasound transducers (hereinafter referred to as cMUTs) have attracted attention. cMUTs are among a category of devices that are referred to as micro electro-mechanical systems (MEMS).

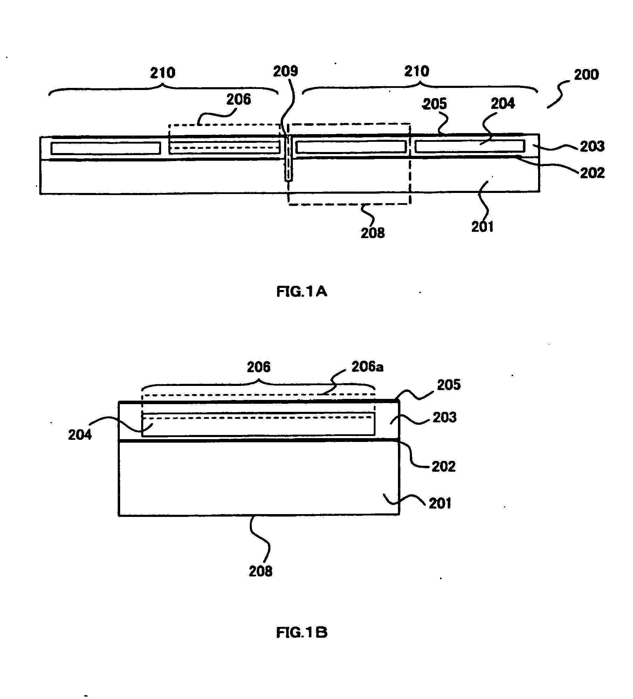

Fig. 1A is a cross-sectional view of a part of a conventional cMUT array. In fig. 1A, the cMUT array includes two transducer elements 210 having a trench 209 between them. The transducer element is the smallest unit for inputting and outputting driving control signals. This transducer element is made of a plurality of transducer cells 208.

The transducer element 210 includes a silicon substrate 201, a bottom electrode 202, a membrane supporting member 203, a cavity 204, and a membrane 206. A unit that includes one cavity 204 is referred to as a transducer cell 208.

Fig. 1B is an enlarged view showing the transducer cell 208. In fig. 1B, the bottom electrode 202 is formed on the silicon substrate 201, and the membrane 206 is supported by the membrane supporting member 203. An upper electrode 205 is formed on the membrane 206. The periphery of the membrane 206 is supported by the membrane supporting member 203.

When voltage is applied between the upper electrode 205 and the bottomelectrode 202 of the transducerelement 210, the membranes 206 of the respective transducer cells 208 simultaneously start the bending vibrations in phase. Thereby, ultrasound is transmitted.

By providing the trench 209, crosstalk between the transducer elements consisting of a plurality of the transducer cells connected with each other is suppressed. Also, an ultrasound damping material may be formed in the trench 209. Also, after removing sacrifice layers used for making the cavities, sacrifice layer removal holes are sealed.

Also, in recent years, capsule endoscopes that are swallowed into body cavities in order to obtain images of the body cavities have been realized (for example, Patent Document 8). Also, a system in which a capsule having a cMUT for making ultrasound diagnoses on body tissues has been disclosed by the applicant of the present application (for example, Patent Documents 9 and 10). By using this ultrasound diagnosis medical capsule endoscope, it is possible to make ultrasound diagnoses at sites at which it was previously difficult to make diagnoses.

Also, the techniques relating to the present invention are disclosed in, for example, patent documents 1 through 10 and the non-patent document 1 below.

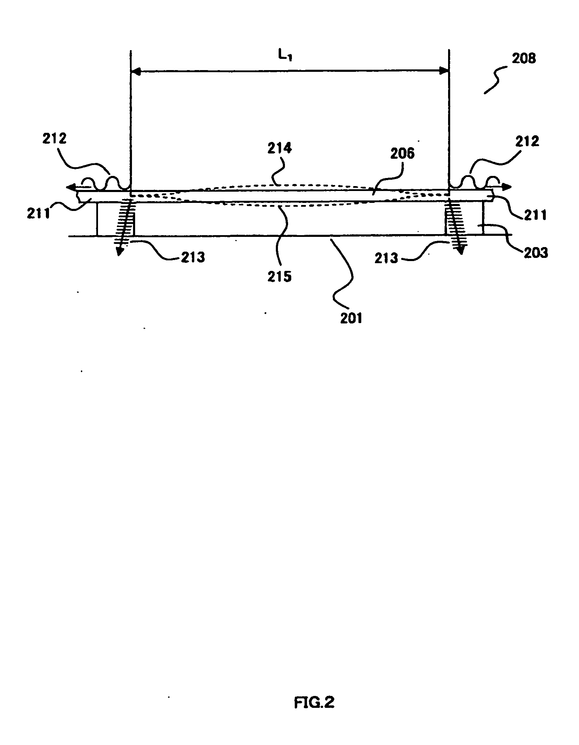

Fig. 2 shows the bending vibration in a conventional cMUT. In a conventional smut, the membranes 206 of the respective transducer cells 208 are continuously arranged in the plane direction (two-dimensional direction). When the membranes 206 are caused to vibrate by driving this type of a cMUT, bending vibrations are caused that can be expressed by a maximum bending deformation 214 caused immediately after the wave of condensation and by a maximum bending deformation 215 caused immediately after the wave of rarefaction.

In this case, the membranes of the adjacent cells are connected to each other and accordingly the bending vibrations of one membrane 206 propagate to another membrane of the adjacent cell so that a portion of the ultrasound vibrations dissipate (expressed as leaky waves 212 in Fig. 2) , and thereby the output sound pressure decreases.

Also, the membrane 206 is supported and fixed by the membrane supporting member 203 at the membrane periphery. This configuration causes a portion of the bending vibrations of the membrane 206 to be converted into longitudinal waves, and the longitudinal waves propagate via the membrane supporting member 203 to a semiconductor substrate 201 as longitudinal waves so that a portion of the ultrasound vibrations dissipate (expressed as supporting member propagation leaky longitudinal waves 213 in Fig. 2), and thereby the output sound pressure decreases.
Patent Document 1: Japanese Patent Application Publication No. 7-274287
Patent Document 2: Japanese Patent Application Publication No. 8-274573
Patent Document 3: Japanese Patent Application Publication No. 2004-274756
Patent Document 4: United States Patent No. 6262946
Patent Document 5: United States Patent No. 6328696
Patent Document 6: United States Patent No. 6328697
Patent Document 7: Publication in Japan of translation of PCT International
Patent Application: No. 2004-503313 (WO 2001/097562)
Patent Document 8: Japanese Patent Application Publication No. 2004-350705
Patent Document 9: Japanese Patent Application Publication No. 2004-350704
Patent Document 10: Japanese Patent Application Publication No. 2004-350705
Non-Patent Document 1: Pages 149 through 152 in the Twelfth version of Volume 1 of "Onkyo Kogaku Genron" written by Tsuyoshi Itoh and published by CORONA PUBLISHING CO., LTD, on December 10, 1980

The article "Finite Element Modelling of Capacitor Micromachined Ultrasonic Transducers", by Yongrae Roth et al., 2000 IEEE Ultrasonics Symposium, pages 905 - 908, discloses a Micromachined ultrasound transducer according to the preamble of claim 1.

### Disclosure of the Invention

An ultrasound transducer according claim 1 is provided. The ultrasound transducer is a micromachined ultrasound transducer and includes a plurality of ultrasound transducer cells that each include a substrate on which a bottom electrode is formed and a membrane provided apart from the substrate and having an upper electrode formed thereon, said ultrasound transducer generating ultrasound with the membrane vibrating when voltage is applied between the bottom electrode and the upper electrode, comprising:
vibration propagation suppression means suppressing propagation of vibration of said each ultrasound transducer cell to an adjacent ultrasound transducer cell.

A method of producing an ultrasound transducer according to claim 18 is provided. The method of producing the ultrasound transducer is a method of producing a micromachined ultrasound transducer including a plurality of ultrasound transducer cells each of which includes a substrate on which a bottom electrode is formed, and a membrane provided apart from the substrate and having an upper electrode formed thereon, said ultrasound transducer generating ultrasound with the membrane vibrating when voltage is applied between the bottom electrode and the upper electrode, and the method comprises the steps of:
providing a supporting member for supporting the membrane along nodes of a fundamental vibration caused when the membrane vibrates freely.

### Brief Description of the Drawings

Fig. 1A is a cross-sectional view of a part of a conventional cMUT array;
Fig. 1B is a cross-sectional view of a conventional cMUT cell;
Fig. 2 shows the bending vibration in a conventional cMUT;
Figs. 3 show the concept of the cMUT cell according to the present invention;
Fig. 4 is a cross-sectional view of a cMUT according to the first embodiment;
Fig. 5 is a cross-sectional view of a cMUT according to the second embodiment;
Fig. 6 is a top view of a cMUT 11 shown in Fig. 5;
Fig. 7 is a cross-sectional view along the line Aa-Ab of Fig. 6;
Fig. 8 is a cross-sectional view along the line Ba-Bb of Fig. 6;
Fig. 9 shows a variation example of the cMUT according to the second embodiment;
Fig. 10 is a cross-sectional view of a cMUT according to the third embodiment;
Fig. 11 is a cross-sectional view of a cMUT according to the fourth embodiment;
Fig. 12 shows a variation example of the fourth embodiment;
Fig. 13 is a cross-sectional view of a cMUT according to the fifth embodiment;
Fig. 14 shows a variation example of the fifth embodiment;
Fig. 15 is a cross-sectional view of a cMUT according to the sixth embodiment;
Fig. 16A is a top view of a bottom electrode according to the sixth embodiment;
Fig. 16B shows deformation of a membrane 15 vibrating when voltage is applied to a bottom electrode 80 in the sixth embodiment;
Fig. 17A is a top view of a conventional bottom electrode 90 shown for the comparison with the bottom electrode in the sixth embodiment;
Fig. 17B is a view showing deformation of the upper electrode 15 vibrating when voltage is applied to a conventional bottomelectrode 90 shown for the comparison with the bottom electrode according to the sixth embodiment;
Fig. 18A shows a first production process for the cMUT according to a seventh embodiment; and
Fig. 18B shows a second production process for the cMUT according to the seventh embodiment.

### Best Modes for Carrying Out the Invention

As was explained in fig. 2, the leaky waves 212 and 213 that have dissipated affect, as crosstalk, the ultrasound transmitted and received through the adjacent transducer cells. The crosstalk between the transducer cells constituting the transducer elements causes variations in the source sound pressure and in the phases of the vibration among the respective transducer elements, and accordingly when the phased array scanning is performed, noise is caused in ultrasound diagnosis images and thereby the ultrasound diagnosis images can be deteriorated.

As described above, because the membrane 206 is supported by the membrane supporting member 203 at the periphery of the membrane, vibrations dissipate so that the mechanical quality factor Q is lowered. This lowered level of the mechanical quality factor Q causes a lowered output of ultrasound and lowered wide band characteristics.

A state in which there is no vibration loss and vibration continues efficiently in a particular range (time axis) corresponds to a high mechanical quality factor Q. In other words, if the dissipation of vibration is made smaller, Q increases. The vibration amplitude at this resonance frequency is Q times the vibration amplitude at a non-resonance frequency. The vibration loss of the membrane is reduced and Q is made greater so that the vibration amplitude is made greater; as a result, great ultrasound transmission sound pressure is obtained.

The present invention provides an ultrasound transducer in which the efficiency of the membrane vibration is increased and the ultrasound transmission efficiency is increased.

A cMUT according to the present invention is formed on a semiconductor substrate. In the present invention, a unit in which a bottom electrode for applying driving control signals and a membrane including an upper electrode serving as a contact electrode are opposed to each other and have a cavity between them is referred to as a cMUT cell. A group of arrays that is the minimum unit made by arranging a large number of the cMUT cells for inputting and outputting the driving control signals is referred to as a cMUT element. A thing in which a large number of the cMUT elements are arrayed is referred to as an array type transducer.

The membrane used in this cMUT cell is divided over the entirety of the periphery between the adjacent cMUT cells, and is independent from the membranes in the adjacent cells. Further, the supporting positions of the membrane supporting members are provided along positions that serve as nodes when the membranes cause the free vibrations.

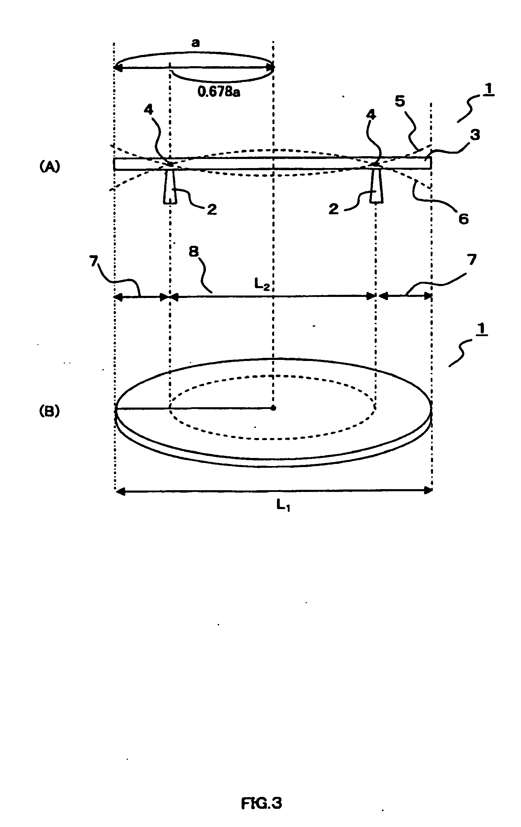

Fig. 3 shows the concept of a cMUT cell according to the present invention. Fig. 3 (A) is a cross-sectional view, and Fig. 3(B) is a perspective view. In Fig. 3, the cMUT cell 1 includes the membrane 3 and the membrane supporting member 2.

The diameter (transducer maximum length) of the membrane 3 is represented by L₁, and the distance (nodal circle diameter dimension 8) between the membrane supporting members is represented by L₂. Also, the periphery of the membrane 3 is an area in the shape of a doughnut in which the circle defined by the membrane supporting members (diameter 8) is removed from the membrane 3 (referred to as a membrane node outside area 7).

The membrane supporting member 2 supports the membrane in a configuration of concentric circles that is smaller than the diameter of the membrane 3 (a circle whose diameter is the diameter 8). The supporting position does not suppress the bending vibration of the membrane 3, and when the fundamental vibrations is caused only in the membrane 3, in other words, when free vibration is caused without fixing the ends of the membrane, the membrane 3 causes a bending vibration that is expressed by a maximum bending deformation 5 caused immediately after the wave of condensation is caused and a maximum bending deformation 6 caused immediately after the wave of rarefaction is caused, and nodes 4 that do not vibrate at any time are caused.

These nodes 4 are parts that do not deform due to the fundamental vibration of the membrane 3. Along the positions at which the nodes 4 are caused, the membrane supporting members 2 are provided. When the radius of the membrane 3 is expressed by "a", in the case of the fundamental vibration, the membrane supporting members 2 are provided at points that are 0.678a from the center of the membrane 3 (non patent document 1).

According to non-Patent Document 1, a circular plate whose periphery is free causes to exist a diameter node line that is arranged at intervals equal to those of the node line of the concentric circle when vibrating. This is especially the case when the shape is symmetric, which POISON solved in 1829, and-the minimum vibration thereof causes the node line at 0.678a (a is the radius of the circular plate), and the next behavior is caused at 0.392a and 0.842a.

As described above, because the membrane of each cell is not connected with the membrane of the next cell, the dissipation of the vibration toward the planar direction (adjacent membrane direction) is avoided. Also, the membrane is supported at the positions corresponding to nodes 4 at which deformation due to the fundamental vibration is not caused, and accordingly it is possible to prevent the vibration from leaking to the semiconductor substrate as the longitudinal wave.

Hereinbelow, the embodiments of the present invention will be explained.

### <First embodiment>

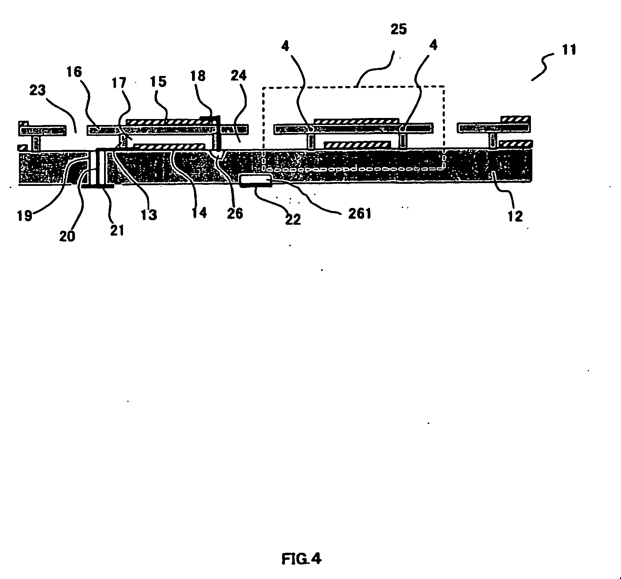

Fig. 4 is a cross-sectional view of a cMUT according to the present embodiment. A cMUT 11 includes a surface-oxidized silicon (Si) substrate 12, membrane supporting member 13, bottom electrodes 14, upper electrodes 15, membranes 16, cavities 17, a ground wire 18, a through hole 19, a through hole wire 20, a signal wire electrode pad 21, and a contact electrode pad 22.

In the present embodiment, each cMUT cell consists of a unit enclosed by a dashed line 25. The cMUT 11 consists of a plurality of cMUT cells 25. Additionally, the dashed line 25 in this unit also includes the ground wire 18, the through hole 19, the through hole wire 20, and the signal wire electrode pad 21, although they are not shown in the figure.

As shown in fig. 3, the membrane 16 is fixed by the membrane supporting members 13 at the positions at which the nodes 4 are caused. The supporting positions are provided at points that are 0.678a from the center of the membrane 16 when the radius of the membrane 16 is expressed by "a" (a is an arbitrary value). The upper electrode 15 is provided on the upper surface of the membrane 16. The membrane supporting members 13 are provided on the upper surface of the silicon substrate 12.

The bottom electrodes 14 are arranged on the surfaces between the membrane supporting members 13 on the surface-oxidized silicon substrate 12. The through hole 19 is provided through the silicon substrate 12, and the through hole wire 20 whose inner wall has undergone the insulation process is provided. The through hole wire 20 and the bottom electrode 14 are electrically continuous to each other. The other end of the through hole wire 20 is electrically continuous to the signal wire electrode pad 21 provided on the silicon substrate 12 whose surface is covered by an insulation film. Thereby, the signal wire electrode pad 21 serves as a terminal on the bottom surface of the silicon substrate 12 with respect to the bottom electrode 14.

The contact electrode pad 22 is an electrode pad for connecting the upper electrode 15 to the GND, and also for causing the upper electrode 15 to be electrically continuous to the bottom surface of the membrane 16. The cavity 17 refers to a space enclosed by the membrane 16 (including the membrane supporting members 13) and the silicon substrate 12 (bottom electrode 14).

By providing diffusion layers (ohmic contact) 26 and 261, it is possible to reduce the resistance between the upper electrode 15 and the contact electrode pad 22 to the minimum level.

Also, the membranes 16 do not contact each other between the adjacent cells 25, and gaps are provided between them. Also, there are spaces (adjacent-cell cavities 24) enclosed by the membrane supporting members 13 and edges of the membranes (membrane node outside area 7) at the lower portions in the spaces between the adjacent cells 25.

The operations of a cMUT 1 will be explained. When voltage is applied between a pair of electrodes of the upper electrode 15 and the bottom electrode 14, the electrodes attract each other, and when the voltage becomes zero, they stop attracting each other. Ultrasound waves are transmitted in the upward direction of the upper electrode 15 when this voltage application is performed at a high speed, and vibrations are activated in the membrane, and thereby ultrasound waves are generated in the membrane 16 on the basis of the vibrations.

According to the present embodiment, the membranes do not contact each other between the adjacent cells, and accordingly the dissipation of the vibrations in the planar direction (direction toward the adjacent membranes) can be avoided. Also, each membrane is supported at the positions of the nodes 4 at which the deformation is not caused by the fundamental vibrations, and accordingly it is possible to prevent the vibrations from leaking to the semiconductor substrate as longitudinal waves.

### <Second embodiment>

In the first embodiment, the membranes do not contact each other between the adjacent cells and there are spaces between them. However, in the present embodiment, a cMUT in which a plurality of trenches are provided in place of the spaces will be explained. Additionally, in the explanations below, the same constituent elements as in the first embodiment are denoted by the same numerals, and the explanations thereof are omitted.

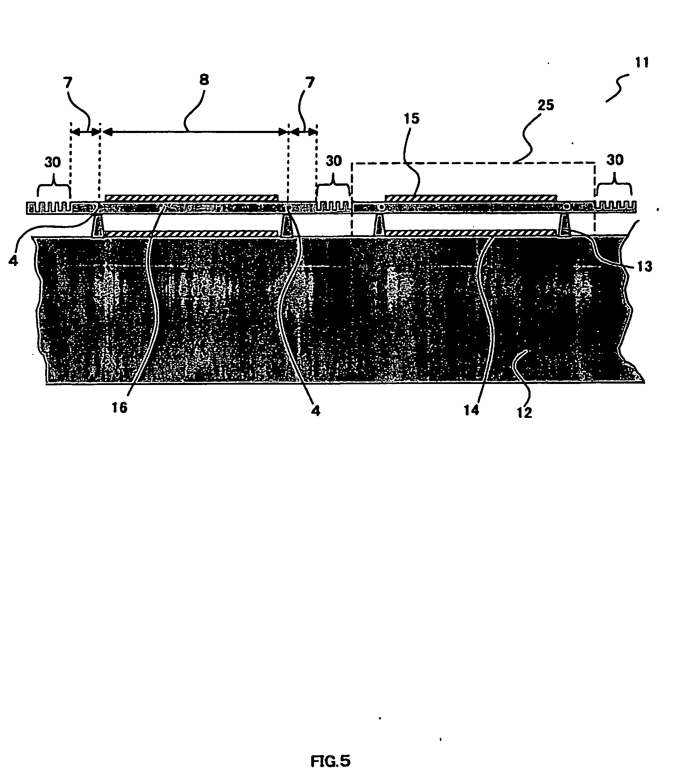

Fig. 5 is a cross-sectional view of the cMUT according to the present embodiment. The configuration shown in fig. 5 is different from that shown in fig. 4 in that gaps are not provided between the membranes in the respective cells, it is configured of one membrane 16, and a plurality of trenches (trench array 30) are provided at the portions corresponding to the gaps in fig. 4. In fig. 5, the ground wire 18, the through hole 19, the through hole wire 20, the signal wire electrode pad 21, and the contact electrode pad 22 are not shown.

In the present embodiment, similarly to the first embodiment, the membrane 16 is supported at points that are 0.678a from the center of the membrane 16 when the radius of the membrane 3 is expressed by "a". However, the positions of nodes may change depending on the number and the depth of trenches. In such a case, it is necessary to determine the positions of the nodes on the basis of experiments.

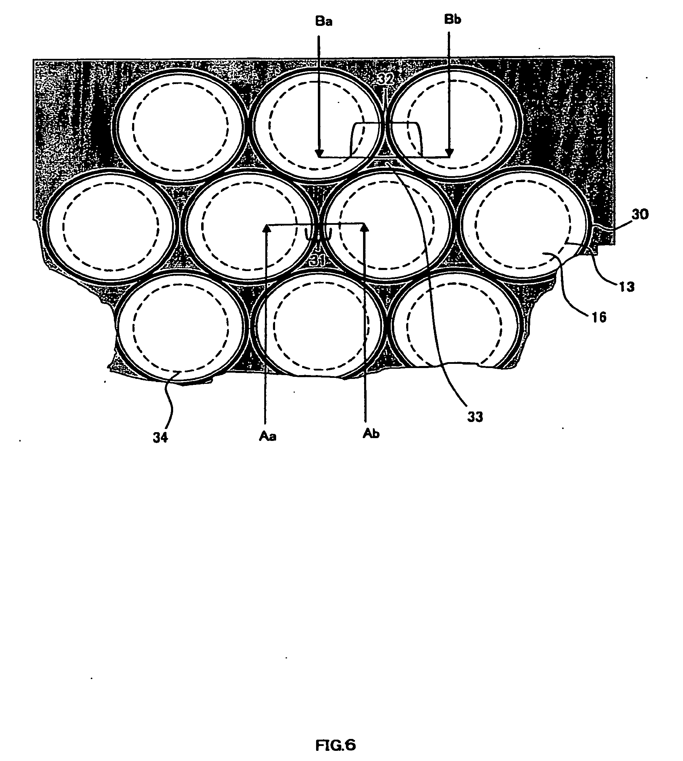

Fig. 6 is a top view of a cMUT 11 shown in fig. 5. The cMUT 11. has a plurality of membranes 16 defined by the trench arrays 30 provided in a circular shape (zonal trench group).

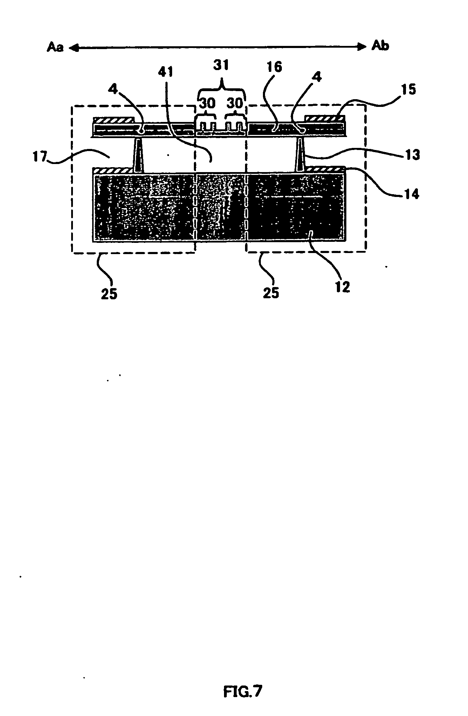

A portion denoted by numeral 31 is a trench array of a portion in which it is the closest to the adjacent cell (hereinafter referred to as a trench array area 31 in which nodes of adjacent-cells are closest). A portion denoted by numeral 32 is an area that is among three adjacent cells. As will be explained in fig. 7, in this area, the thickness of the membrane changes noncontinuously so that the acoustic impedance becomes noncontinuous. Hereinafter, this area is referred to as an acoustic-impedance noncontinuous area 32 among three adjacent cells.

The circles 34 depicted by the dashed lines are portions corresponding to the portions supported by the membrane supporting members 13, and inherently cannot be seen from above because they are below the membranes.

Fig. 7 is a cross-sectional view along the line Aa-Ab of fig. 6. A cavity that is under the membrane 16 and between the adjacent cells is referred to as a lower cavity 41 of acoustic-impedance noncontinuous-region between adjacent cells. The trench array area 31 that is corresponding to the lower cavity 41 in the membrane 16 is configured of a combination of a plane and a plurality of trench arrays 30, and accordingly the thickness of the membrane changes noncontinuously. Thus, the membrane 16 corresponding to this portion causes the noncontinuous acoustic impedance.

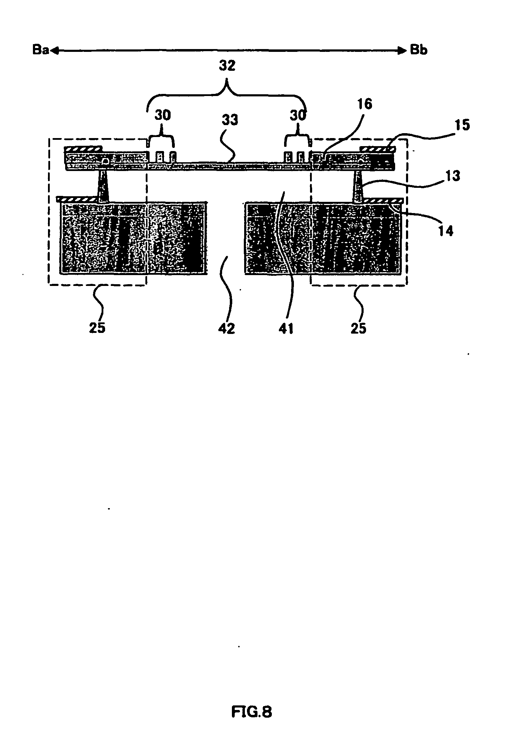

Fig. 8 is a cross-sectional view along the line Ba-Bb of Fig. 6. In fig. 8, there is the acoustic-impedance noncontinuous area 32 between the adjacent trench arrays 30. The portion between the trench arrays 30 has a thickness smaller than that of the other portions (a thin layer area 33).

Also, in the silicon substrate 12; the portion corresponding to the thin layer area 33 has a through hole 42. The through hole 42 is made for the purpose of air ventilation. If a through hole 42 is not present, the lower cavity 41 is sealed, and the dumping effect is caused so that the mechanical quality factor Q decreases.

Also, if the lower cavity 41 is sealed, dumping against the vibrations in the thin layer area 33 is caused because the thin layer area 33 vibrates while synchronizing the vibrations of the membrane 16. This dumping decreases the vibration efficiency of the membrane.

Thugs, air ventilation is realized in the lower cavity 41 via the through hole 42 in order to increase the vibration efficiency of the vibrations in the membrane and to increase the ultrasound wave transmission efficiency so that the lower cavity 41 is prevented from being sealed, and the membrane 16 is caused to vibrate highly efficiently.

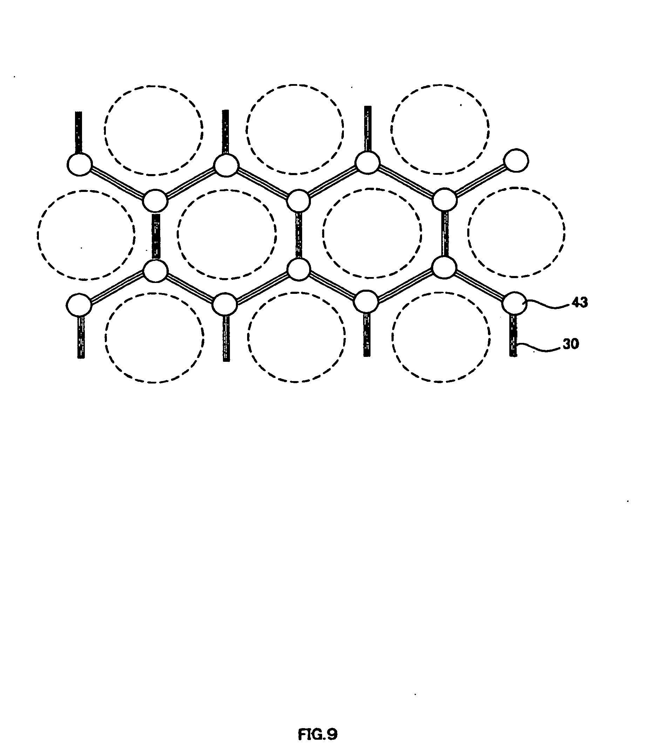

Fig. 9 shows a variation example of fig. 6. Fig. 9 shows a configuration in which the shape formed by the trench array 30 is changed from a circle to a hexagon, and holes 43 that lead to the middle of the membrane in the thickness direction are made at the corners. The holes 43 are made in order to suppress the concentration of forces. The shape of the trench is not limited to a hexagon, and can be other polygons.

According to the present embodiment, a zonal trench group is provided as acoustic isolation means between the ultrasound transducer cells, and thereby the bend (plate waves) in the membrane is enclosed in the cell and does not easily propagate to the membranes of the adjacent cells so that it is possible to suppress the dissipation of the vibration in the plane direction. Also, it is possible to suppress the variation in the ultrasound output caused by the liquid invading through the ultrasound transmission/reception plane because there is no gap between the membranes.

### <Third embodiment>

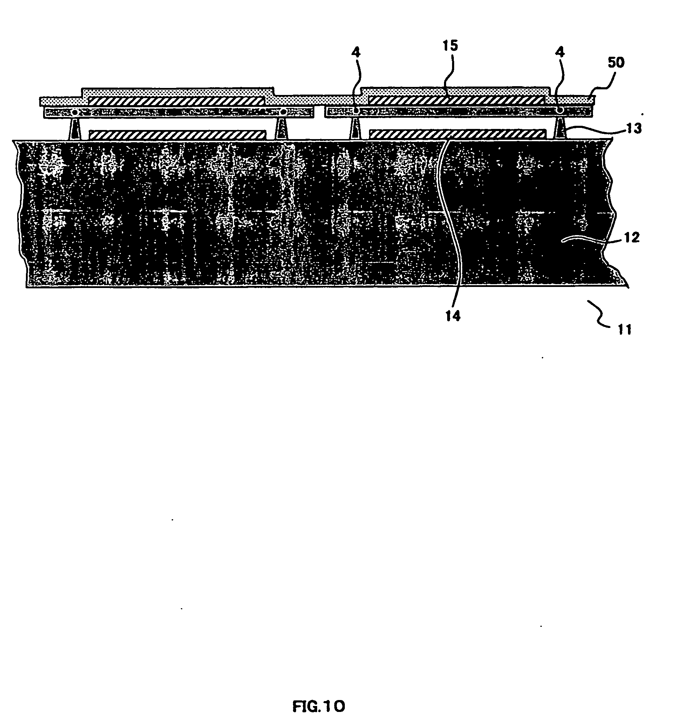

Fig. 10 is a cross-sectional view of a. cMUT according to the present embodiment. Fig. 10 shows a configuration in which the upper surfaces of the membrane 16 and the upper electrode 15 of the cMUT shown in fig. 4 are coated with a protective layer 50.

Examples of the materials that can be used for the protective layer 50 are parylene, polymide, Teflon (registered trademark), Cytop, and the like.

This protective film 50 may be a film made of nanometer-sized particles (nano-coating film). The components of the protective film 50 (product name: x-protect DS 3010; manufacturer name: NANO-X) are silicon (Si), zirconium (Zr), titanium (Ti), oxygen (0), and other organic constituents (polymer compound), and are in a mesh-configuration. This structure is obtained by hydrolyzing metallic alkoxide compound such as silicon (Si), zirconium (Zr), titanium (Ti) or the like. Also, the organic constituents of the base material are present along the mesh all over the film. This structure has a mesh configuration over its entirety; however, a portion in which the organic constituents are not along the mesh of the non-organic constituent can be generated. Therefore, nanoparticles that are free from the mesh can exist. Accordingly, it is possible to select a nanoparticle (also in a mesh-configuration) or a mesh-configuration over the entirety of which there are organic constituents by selecting the production conditions such as the heating condition, the manner of hydrolyzation, the PH adjustment, or the like. Also, the nanometer-sized nonorganic constituent may be one or a plurality of silicon, titanium, and zirconium. Also, the nanometer-sized nonorganic constituent may be one or a plurality of the oxidative products of silicon, the oxidative products of titanium, and the oxidative products of zirconium. The protective films made of these non-organic chemical compounds have excellent corrosion resistance and humidity resistance.

According to an embodiment of the present invention, it is possible to prevent liquid from invading because the gaps between the membranes are sealed by the protective film 50. Also, the present embodiment can be applied in combination with any of the first and second embodiments.

### <Fourth embodiment>

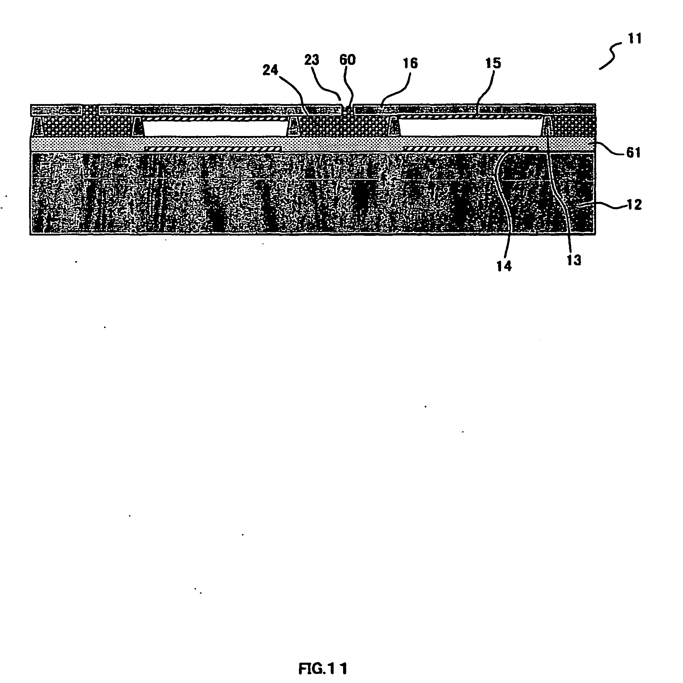

Fig. 11 is a cross-sectional view of a cMUT according to the present embodiment. The configuration shown in fig. 11 is different from the configuration shown in fig. 4 in that the bottom electrode 14 is formed on the upper surface of the silicon substrate 12 (SiO₂/Si) whose surface has undergone the insulation process and an insulation film 61 is formed thereon in the configuration shown in fig. 11. Also, the adjacent-cell cavity 24 and a gap 23 are filled with filler agent 60 so that the entire membrane in the cMUT 11 is flat.

For the insulation film 61, materials having a high dielectric constant such as SrTiO₃ , barium titanate BaTiO₃, barium strontium titanate, tantalum pentoxide, niobium pentoxide stabilized tantalum pentoxide, oxidized aluminum, oxidized titanium TiO₂, or the like can be used.

For the filler agent 60, materials that are flexible to decrease a vibration loss and that have an acoustic impedance greatly different from that of the membrane material are used in order to increase the S/N ratio of the vibration in the membrane 16. For the filler agent 60, resin foam is used. It is desirable that the resin foam have an acoustic impedance greatly different from that of the membrane material.

For the resin foam, such substances as obtained by making SiO₂ or a SiOF film porous can be used. The resin foam is used for the Low-k (insulation film between low dielectric layers) film material. Also, when an organic film is used as the resin foam, plasma CVD films made of polyimide, parylene, or teflon can be used. Also, the coefficient of elasticity becomes one tenth or lower.

Also, for the filler agent 60, porous silicon can be used. Porous silicon is a thing in which countless nano-scale micro pipes are formed in the plate thickness direction of silicon. The acoustic impedance is very small because there is air in the micro capillary tube.

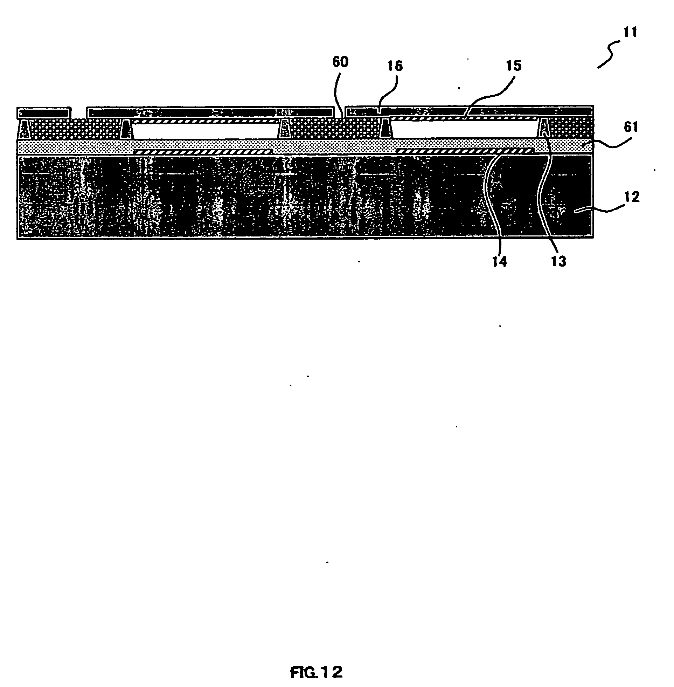

Fig. 12 shows a variation example of the configuration shown in fig. 11. In fig. 12, only the adjacent-cell cavity 24 is filled with the filler agent 60 and the gap 23 is not filled. Thereby, it is possible to increase sensitivity in the ultrasound reception because the membrane 16 is not restrained.

Also, it is desirable that the acoustic impedance of the filler agent 60 filling the gap 23 be equal to or lower than twenty percent of the acoustic impedance of the membrane 16, and more desirable that it be equal to or lower than ten percent. This is because if it exceeds ten percent, the vibration efficiency of the membrane decreases by 3dB, and this affects the image sensitivity (brightness) and increases the crosstalk by 3dB so that the image contrast is affected.

According to the present embodiment, by filling in the gap around the periphery of the membrane with a filling agent, it is possible to suppress the variation of the ultrasound output that would otherwise be caused by the invasion of liquid. Also, the present invention can be applied in combination with any of the first through third embodiments.

### <Fifth embodiment>

In the present embodiment, a cMUT in which the integration density of the ultrasound cells is increased will be explained.

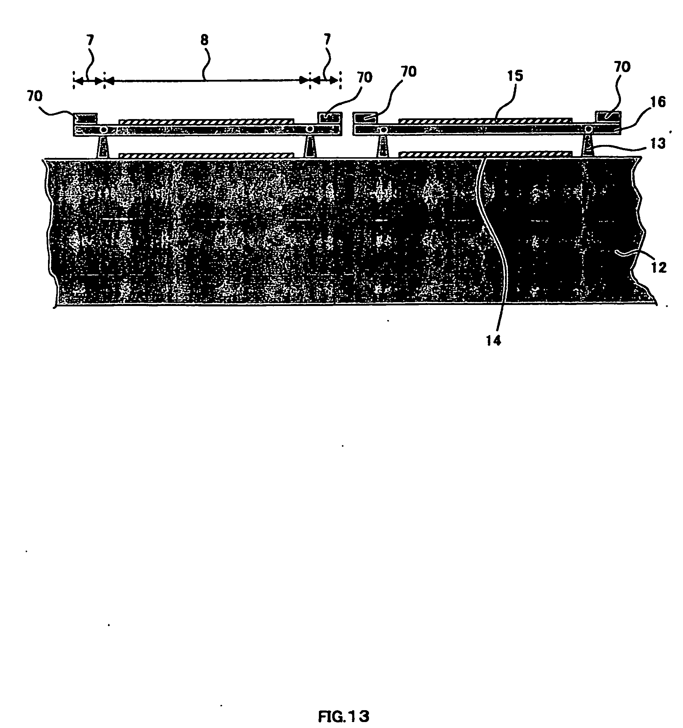

Fig. 13 is a cross-sectional view of a cMUT according to the present embodiment. Fig. 13 shows a thing in which weight members 70 are added to the peripheries (membrane node outside area 7) of the membranes 16 shown in fig. 4. The nodes are shifted to the side of the weight members 70 because the weight members 70 are arranged on the membrane node outside area 7. As a result of this, the width of each membrane node outside area 7 is reduced so that the diameter 8 becomes greater.

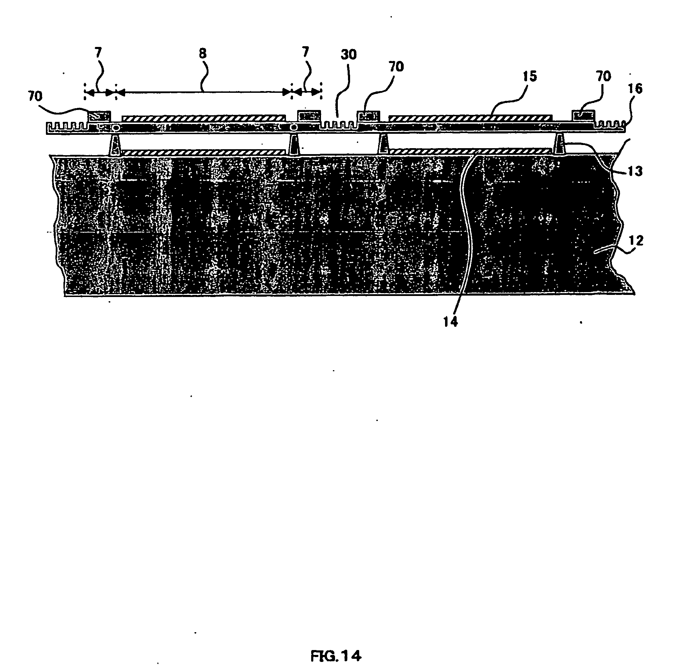

Fig. 14 shows a variation example of the configuration in fig. 13. Fig 14 shows a thing in which the weight members 70 are provided on the membrane node outside area 7 shown in fig. 5.

According to the present embodiment, the nodes shift to the weight members 70 because the weight members 70 are arranged on the membrane node outside area 7, and accordingly the width of each membrane node outside area 7 is reduced and the length of the diameter 8 becomes greater. As a result of this, the ratio of the area of the membrane node outside area 7 (the doughnut shape) to the area of the inner area (a circle defined by the diameter 8) becomes greater.

Accordingly, the area of the circle defined by the diameter 8 becomes greater, and thus it is possible to increase the capacitance that directly relates to the sound pressure generated. Also, as the area of the membrane node outside area 7 is reduced, the intervals between the adjacent cells can be reduced, and thereby it is possible to increase the integration density of the cMUT cells. Thereby, the sensitivity of an ultrasound image increases and S/N also increases due to the reduction of the sidelobe. Also, the present embodiment can be applied in combination with any of the first through fourth embodiments.

### <Sixth embodiment>

In the present embodiment, an ultrasound transducer will be explained in which the shape of the bottom electrode is changed in order to keep the shape of the vibrating membrane as flat as possible.

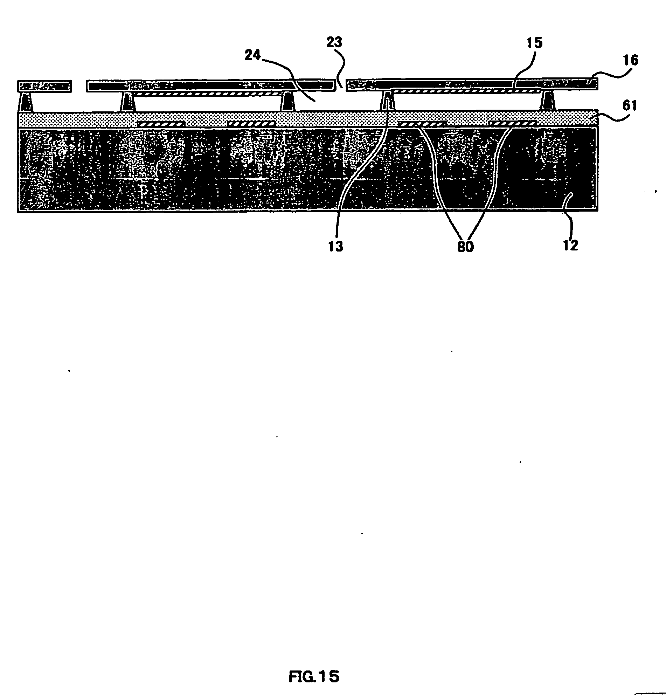

Fig. 15 is a cross-sectional view showing a cMUT according to the present embodiment. In Fig. 15, bottom electrodes 80 are formed on the upper surface of the silicon substrate (SiO₂/Si), and an insulation film 61 is formed thereon.

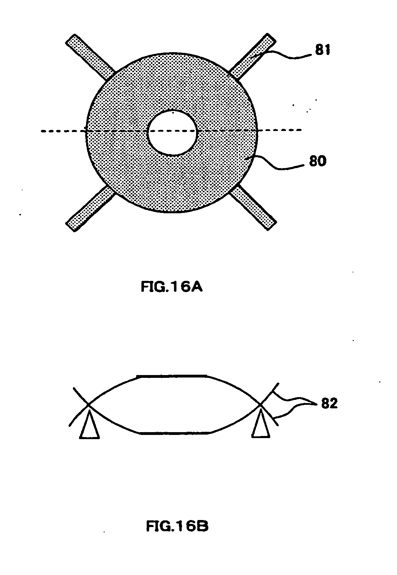

Fig. 16A is a top view of the bottom electrode 80 in the present embodiment. When the bottom electrode 80 is viewed from above, it is in the shape of a doughnut, and wires 81 are provided at the four points in order to be connected to the adjacent electrodes.

Numeral 82 in fig. 16B denotes the manner of the deformation of the upper electrode 15 vibrating when voltage is applied to the bottom electrode 80. It is sufficient to make one of the upper and the bottom electrodes be in the shape of a doughnut; accordingly, the bottom electrode is in the shape of a doughnut and the upper electrode 15 is circular. Also, the upper electrode on the GND side has the effect of reducing the noise arriving from outside circumstances, and accordingly it is desirable that the bottom electrode on the signal side be in the shape of a doughnut.

As shown in fig. 16B, no electrostatic force is applied between the central portion of the bottom electrode 80 at which there is no electrode and the corresponding central portion of the upper electrode 15, and accordingly that portion of the upper electrode 15 does not deform so that it can be substantially flat.

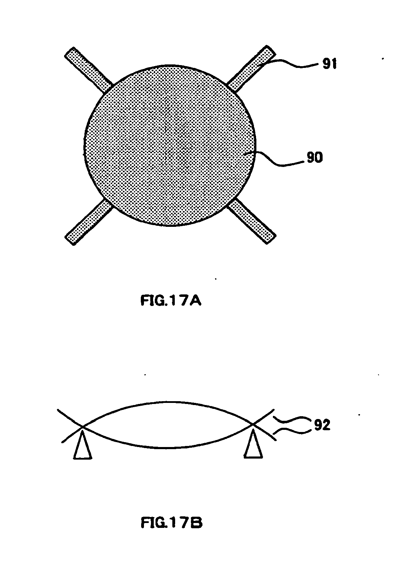

A conventional bottom electrode will be explained by referring to Figs. 17A and 17B in order to explain the present embodiment more clearly. When viewed from above, a conventional bottom electrode 90 is circular, and wires 91 are provided at the four points in order to be connected to the adjacent electrodes (Fig. 17A). As described above, the conventional bottom electrode 90 is an electrode in its entirety.

As shown in fig. 17B, when voltage is applied to the bottom electrode 14, the electrostatic force is applied also to the central portion of the upper electrode 15, and accordingly the central portion of the upper electrode 15 cannot remain flat, and thus deforms to a non-linear shape as denoted by numeral 92. As described above, the non-linear vibrations affect operations such as the harmonic imaging in which non-linear characteristics are utilized.

The present embodiment can be applied in combination with any of the first and fifth embodiments.

According to the present embodiment, the central portion of the upper electrode corresponding to the portion in which there is no bottom electrode is kept substantially flat and is not deformed, and accordingly it is possible to apply a greater voltage and generate greater power.

### <Seventh Embodiment>

In the present embodiment, a method of producing a cMUT will be explained. There are two methods (the bulk machining method and the surface micromachining method) of producing a cMUT, and in each method the supporting member is provided further inward than the periphery and the supporting member is sealed in an air-tight manner so as to increase the deformation amount of the membrane. In the present embodiment, the bulk machining method will be explained.

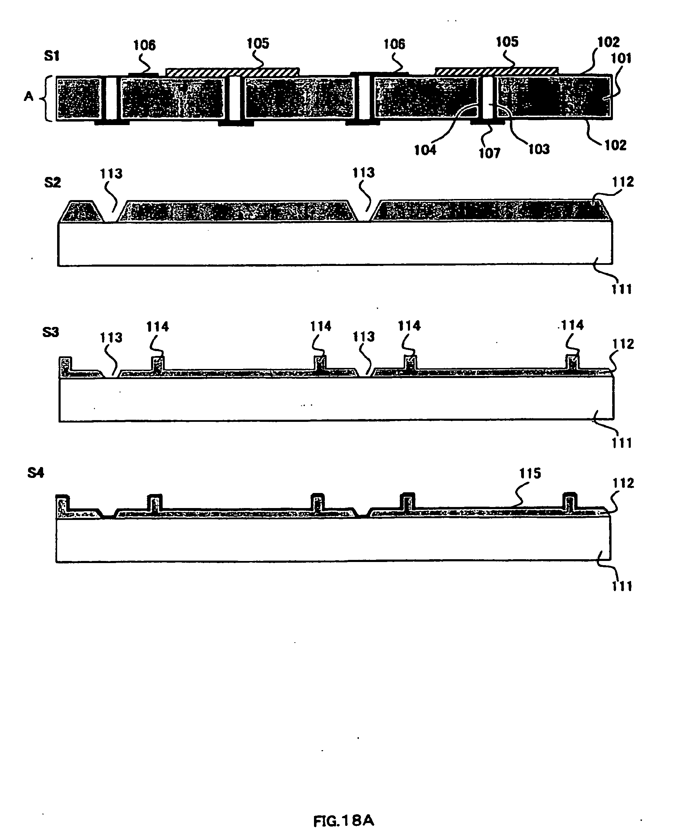

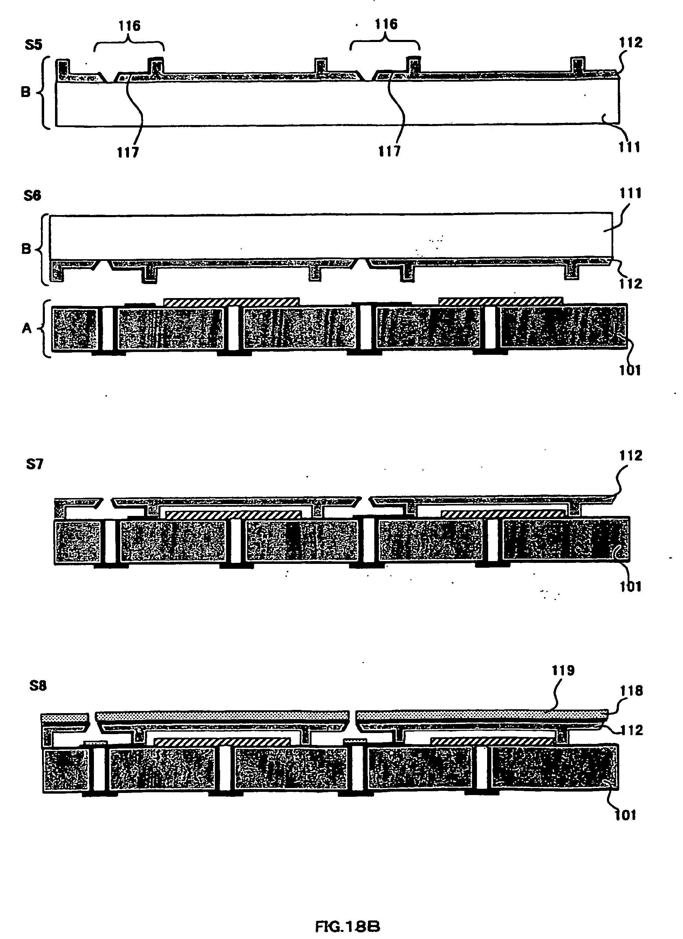

Figs. 18A and 18B show the steps for producing a cMUT according to the present embodiment.

First, the surface of an N-type silicon substrate 101 (with a thickness between about 100p and a bout500µ) is masked with an oxide film (SiO₂) 102. An oxide film with a thickness between about 3000 angstroms and about 4000 angstroms is formed by using the wet oxidization method. Then, the patterning is performed in order to form through-hole electrodes 104 for bottom electrodes by using the photolithography process, and the oxide film that has undergone the patterning is removed in the etching step.

Next, holes 103 are made by performing ICP-RIE (Inductively Coupled Plasma Reactive Ion Etching) at portions that are not masked.

Next, an electrode film (Pt/Ti) is formed on the upper and lower surfaces of the silicon substrate 101 and the inner wall surface of the holes 103. Bottom electrodes 105, electrodes-for-wiring 106, and electrode pads 107 are formed (step 1; a step is referred to as "S" hereinafter) after performing the patterning step and the etching step on the electrode film. The material of the electrodes is not limited to Pt/Ti, and Au/Cr, Mo, W, phosphor bronze, Al, and the like may be used. The structure made in S1 is referred to as wafer A.

Next, another Si substrate 111 is prepared, and an oxide film SiO₂ 112 is formed by oxidizing one of upper and lower surfaces of the substrate 111. An etching process (etching process using, for example, the CVD method) is performed on a part of the oxide film 112 so that it is tapered by the ICP-RIE (S2) . The portions formed through this etching process are referred to as concaves 113.

Next, membrane supporting members 114 are formed. Similarly to S2, the etching process is performed on the portions other than the membrane supporting members on the oxide film 112 by using ICP-RIE (S3) . This etching process is performed in such a manner that an oxide film 112 of the thickness of the membrane is not etched. Also, the portions between the two concaves 113 on the oxide film 112 will serve as the membrane, and accordingly the etching process is performed in such a manner that the membrane supporting members 114 are formed along points that are 0.678a from the center of each membrane (a is the radius of the membrane).

Next, an electrode film 115 is formed on the surface of the oxide film (S4) . Thereafter, a patterning process is performed in order to remove the portions other than the portions denoted by numeral 116 on the electrode film (S5). Thereby, electrodes 117 for wires are formed. The structure produced in this S4 is referred to as wafer B.

Next, wafer A and wafer B are jointed to each other (S6). Thereafter, a silicon substrate 111 is removed by performing the etching process so that the oxide film 112 is exposed (S7). This etching process can be performed by using, for example, TMAH (tetramethylammonium hydroxide) or the like.

Next, an electrode film 118 is formed on the exposed oxide film 112, and a protective film (SiN) 119 is formed thereon (S8).

Thereby, it is possible to produce an ultrasound transducer in which membrane supporting members are provided at the nodes that are caused when membranes vibrate freely. Also, in the present embodiment, a method of producing a cMUT according to the first embodiment has been explained as an example. However, cMUTs according to the other embodiments can be produced similarly by using the micromachining process.

Also, the scope of the present invention is not limited to any of the above described embodiments, and it is possible to employ various configurations or shapes.

The first through seventh embodiments can be combined in various combinations.

Also, the ultrasound transducer according to the present invention can be included in an endoscopic ultrasound diagnosis system as an ultrasound endoscope, a miniature ultrasound probe, an in-vessel ultrasound probe, or an ultrasound capsule endoscope.

According to the present invention, the respective membranes are independent from the membranes of the adj acent cells so that it is possible to avoid dissipation of the vibrations in the plane direction.

Also, according to the present invention, the membranes are supported at the nodes, which do not vibrate inherently, so that the vibrations of the membranes do not leak via the supporting members (in other words, vibration loss of the membranes does not occur). Accordingly, a high mechanical quality factor Q is obtained. As a result of this, the ultrasound transmission efficiency increases.

Also, according to the present invention, the vibrations of the membranes do not leak via the supporting members, and accordingly crosstalk can be avoided, whereby vibrations that would leak via supporting members become the longitudinal waves, the longitudinal waves are reflected by the back plane of the silicon substrate, and the reflected waves are converted into membrane vibrations of the adjacent membrane cells through the supporting members of the adjacent cells.

## Claims

1. A micromachined ultrasound transducer including a plurality of ultrasound transducer cells (1, 25) each of which includes a substrate (12) on which a bottom electrode (14, 80) is formed, a membrane (3, 16) provided apart from the substrate (12) and having an upper electrode (15) formed thereon, and a supporting member (2, 13) provided on the substrate (12) for supporting the membrane (3, 16), said ultrasound transducer generating ultrasound with the membrane (3, 16) vibrating when voltage is applied between the bottom electrode (14, 80) and the upper electrode (15), comprising:
vibration propagation suppression means suppressing propagation of vibration of said each ultrasound transducer cell (1, 25) to an adjacent ultrasound transducer cell (1, 25) or to the membrane supporting member (2, 13) **characterized in that**:
the vibration propagation suppression means comprise supporting members (2, 13) provided, in order to support the membrane, along nodes (4) caused when fundamentally free vibrations are caused in the membrane (3, 16).

2. The ultrasound transducer according to claim 1, wherein:
the vibration propagation suppression means further comprise a trench (30, 31) provided between membranes (2, 13) of the adjacent ultrasound transducer cells (1, 25).

3. The ultrasound transducer according to claim 1, wherein:
the vibration propagation suppression means further comprises an acoustic isolation means (41, 60) for acoustically isolating said each ultrasound transducer cell (1, 25) from the adjacent ultrasound transducer cells (1, 25).

4. The ultrasound transducer according to claim 3, wherein:
the acoustic isolation means has an acoustic impedance equal to or lower than twenty percent of an acoustic impedance of the membrane (3, 16).

5. The ultrasound transducer according to claim 4, wherein:
the acoustic isolation means (41) is a single air layer.

6. The ultrasound transducer according to claim 4, wherein:
the acoustic isolation means is a solid material including gas.

7. The ultrasound transducer according to claim 6, wherein:
the solid material is porous silicon.

8. The ultrasound transducer according to claim 6, wherein:
the solid material is resin foam.

9. The ultrasound transducer according to claim 1, wherein:
an outer shape of a membrane (3, 16) of each ultrasound transducer cell (1, 25) isolated by the vibration propagation suppression means is circular or polygonal.

10. The ultrasound transducer according to claim 9, wherein:
a shape of the supporting member (2, 13) supporting the membrane is similar to the outer shape.

11. The ultrasound transducer according to claim 1, comprising:
mass loading means (70) at a position further outward than positions of the nodes (4) on the membrane (3, 16).

12. The ultrasound transducer according to claim 1, wherein:
a surface of a side for transmitting and receiving the ultrasound is coated with a nano-coating film (50) .

13. An endoscopic ultrasound diagnosis system including the ultrasound transducer according to claim 1.

14. The endoscopic ultrasound diagnosis system according to claim 13, wherein:
the endoscopic ultrasound diagnosis system is an ultrasound endoscope.

15. The endoscopic ultrasound diagnosis system according to claim 13, wherein:
the ultrasound transducer is adapted to be used as an ultrasound probe inserted into an instrument channel in an endoscope.

16. The endoscopic ultrasound diagnosis system according to claim 13, wherein:
the ultrasound transducer is adapted to be used as an in-vessel ultrasound probe.

17. The endoscopic ultrasound diagnosis system according to claim 13, wherein:
the ultrasound transducer is adapted to be used as an ultrasound capsule endoscope.

18. A method of producing a micromachined ultrasound transducer including a plurality of ultrasound transducer cells (1, 25) each of which includes a substrate (12) on which a bottom electrode (14, 80) is formed, and a membrane (3, 16) provided apart from the substrate (129 and having an upper electrode (15) formed thereon, said ultrasound transducer generating ultrasound with the membrane (3, 16) vibrating when voltage is applied between the bottom electrode (14, 80) and the upper electrode (15), the method **characterized by** comprising:
providing a supporting member (2, 13) on the substrate for supporting the membrane (3, 16) along nodes (4) caused when fundamentally free vibrations are caused in the membrane, in order to suppress propagation of vibration of said each ultrasound transducer cell (1, 25) to an adjacent ultrasound transducer cell (1, 25) or to a membrane supporting member (2, 13).

## Patentansprüche

1. Mikromechanisch hergestellter Ultraschallwandler mit mehreren Ultraschallwandlerzellen (1, 25), von denen jede ein Substrat (12), auf dem eine untere Elektrode (14, 80) ausgebildet ist, eine Membran (3, 16), die getrennt von dem Substrat (12) vorgesehen ist und die eine darauf ausgebildete obere Elektrode (15) hat, sowie ein auf dem Substrat (12) vorgesehenes Halterungselement (2, 13) zum Haltern der Membran (3, 16) aufweist, wobei der Ultraschallwandler mit der Membran (3, 16), die vibriert, wenn Spannung zwischen der unteren Elektrode (14, 80) und der oberen Elektrode (15) angelegt wird, Ultraschall erzeugt, mit:
Vibrationsausbreitungs-Unterdrückungsmitteln, die eine Ausbreitung von Vibration von jeder Ultraschallwandlerzelle (1, 25) zu einer benachbarten Ultraschallwandlerzelle (1, 25) oder zu dem Membran-Halterungselement (2, 13) unterdrücken, **dadurch gekennzeichnet, dass**:
die Vibrationsausbreitungs-Unterdrückungsmittel Halterungselemente (2, 13) umfassen, die vorgesehen sind zur Halterung der Membran (3, 16) entlang Knoten (4), welche entstehen, wenn im Wesentlichen freie Vibrationen in der Membran (3, 16) hervorgerufen werden.

2. Ultraschallwandler nach Anspruch 1, wobei:
die Vibrationsausbreitungs-Unterdrückungsmittel ferner einen zwischen Membranen (2, 13) der benachbarten Ultraschallwandlerzellen (1, 25) vorgesehenen Trenngraben (30, 31) umfassen.

3. Ultraschallwandler nach Anspruch 1, wobei:
die Vibrationsausbreitungs-Unterdrückungsmittel ferner ein akustisches Isolationsmittel (41, 60) zum akustische Isolieren jeder Ultraschallwandlerzelle (1, 25) von den benachbarten Ultraschallwandlerzellen (1, 25) umfassen.

4. Ultraschallwandler nach Anspruch 3, wobei:
das akustische Isolationsmittel eine akustische Impedanz gleich oder kleiner als zwanzig Prozent einer akustischen Impedanz der Membran (3, 16) aufweist.

5. Ultraschallwandler nach Anspruch 4, wobei:
das akustische Isolationsmittel (41) eine einzelne Luftschicht ist.

6. Ultraschallwandler nach Anspruch 4, wobei:
das akustische Isolationsmittel ein Gas enthaltendes Feststoffmaterial ist.

7. Ultraschallwandler nach Anspruch 6, wobei:
das Feststoffmaterial poröses Silizium ist.

8. Ultraschallwandler nach Anspruch 6, wobei:
das Feststoffmaterial Harzschaum ist.

9. Ultraschallwandler nach Anspruch 1, wobei:
eine äußere Form einer Membran (3, 16) jeder von dem Vibrationsausbreitungs-Unterdrückungsmittel isolierten Ultraschallwandlerzelle (1, 25) kreisförmig oder polygonal ist.

10. Ultraschallwandler nach Anspruch 9, wobei:
eine Form des die Membran halternden Halterungselements (2, 13) ähnlich der äußeren Form ist.

11. Ultraschallwandler nach Anspruch 1, mit:
Masse-Lademitteln (70) an einer Position, die sich weiter außen befinden als Positionen der Knoten (4) an der Membran (3, 16).

12. Ultraschallwandler nach Anspruch 1, wobei:
eine Oberfläche einer Seite zum Übertragen und Empfangen des Ultraschalls mit einem Nano-Beschichtungsfilm (50) beschichtet ist.

13. Endoskopisches Ultraschall-Diagnosesystem, das den Ultraschallwandler nach Anspruch 1 aufweist.

14. Endoskopisches Ultraschall-Diagnosesystem nach Anspruch 13, wobei:
das endoskopische Ultraschall-Diagnosesystem ein Ultraschall-Endoskop ist.

15. Endoskopisches Ultraschall-Diagnosesystem nach Anspruch 13, wobei:
der Ultraschallwandler als eine in einen Instrumentenkanal in einem Endoskop eingesetzte Ultraschallsonde verwendet zu werden vermag.

16. Endoskopisches Ultraschall-Diagnosesystem nach Anspruch 13, wobei:
der Ultraschallwandler als eine gefäßinterne Ultraschallsonde verwendet zu werden vermag.

17. Endoskopisches Ultraschall-Diagnosesystem nach Anspruch 13, wobei:
der Ultraschallwandler als ein Ultraschall-Kapselendoskop verwendet zu werden vermag.

18. Verfahren zur Herstellung eines mikromechanisch hergestellten Ultraschallwandlers mit mehreren Ultraschallwandlerzellen (1, 25), von denen jede ein Substrat (12), auf dem eine untere Elektrode (14, 80) ausgebildet ist, sowie eine Membran (3, 16), die getrennt von dem Substrat (12) vorgesehen ist und die eine darauf ausgebildete obere Elektrode (15) hat, aufweist, wobei der Ultraschallwandler mit der Membran (3, 16), die vibriert, wenn Spannung zwischen der unteren Elektrode (14, 80) und der oberen Elektrode (15) angelegt wird, Ultraschall erzeugt, wobei das Verfahren **dadurch gekennzeichnet ist, dass** es umfasst:
Bereitstellen eines Halterungselement (2, 13) auf dem Substrat zum Haltern der Membran (3, 16) entlang Knoten (4), die entstehen, wenn im Wesentlichen freie Vibrationen in der Membran hervorgerufen werden, um eine Ausbreitung von Vibration von jeder Ultraschallwandlerzelle (1, 25) zu einer benachbarten Ultraschallwandlerzelle (1, 25) oder zu einem Membran-Halterungselement (2, 13) zu unterdrücken.

## Revendications

1. Transducteur ultrasonore micro-usiné incluant une pluralité de cellules (1, 25) de transducteur ultrasonore, chacune desquelles inclut un substrat (12) sur lequel une électrode inférieure (14, 80) est formée, une membrane (3, 16) prévue à l'écart du substrat (12) et ayant une électrode supérieure (15) formée sur celle-ci, et un élément support (2, 13) prévu sur le substrat (12) pour supporter la membrane (3, 16), ledit transducteur ultrasonore générant un ultrason avec la membrane (3, 16) vibrant lorsqu'une tension est appliquée entre l'électrode inférieure (14, 80) et l'électrode supérieure (15), comprenant:
un moyen de suppression de propagation de vibration supprimant une propagation de la vibration de chaque dite cellule (1, 25) de transducteur ultrasonore jusqu'à une cellule (1, 25) de transducteur ultrasonore adjacente ou jusqu'à l'élément support (2, 13) de membrane,
**caractérisé en ce que**:
le moyen de suppression de propagation de vibration comprend des éléments support (2, 13) prévus, afin de supporter la membrane, le long de noeuds (4) causés lorsque des vibrations fondamentalement libres sont causées dans la membrane (3, 16).

2. Transducteur ultrasonore selon la revendication 1, dans lequel:
le moyen de suppression de propagation de vibration comprend en outre une tranchée (30, 31) prévue entre des membranes (2, 13) des cellules (1, 25) de transducteur ultrasonore adjacentes.

3. Transducteur ultrasonore selon la revendication 1, dans lequel:
le moyen de suppression de propagation de vibration comprend en outre un moyen d'isolation acoustique (41, 60) pour isoler acoustiquement chaque dite cellule (1, 25) de transducteur ultrasonore des cellules (1, 25) de transducteur ultrasonore adjacentes.

4. Transducteur ultrasonore selon la revendication 3, dans lequel:
le moyen d'isolation acoustique a une impédance acoustique égale ou inférieure à vingt pour cent d'une impédance acoustique de la membrane (3, 16).

5. Transducteur ultrasonore selon la revendication 4, dans lequel:
le moyen d'isolation acoustique (41) est une couche d'air unique.

6. Transducteur ultrasonore selon la revendication 4, dans lequel:
le moyen d'isolation acoustique est un matériau solide incluant un gaz.

7. Transducteur ultrasonore selon la revendication 6, dans lequel:
le matériau solide est un silicium poreux.

8. Transducteur ultrasonore selon la revendication 6, dans lequel:
le matériau solide est une mousse de résine.

9. Transducteur ultrasonore selon la revendication 1, dans lequel:
une forme extérieure d'une membrane (3, 16) de chaque cellule (1, 25) de transducteur ultrasonore isolée par le moyen de suppression de propagation de vibration est circulaire ou polygonale.

10. Transducteur ultrasonore selon la revendication 9, dans lequel:
une forme de l'élément support (2, 13) supportant la membrane est similaire à la forme extérieure.

11. Transducteur ultrasonore selon la revendication 1, comprenant:
un moyen de chargement de masse (70) en une position plus vers l'extérieur que des positions des noeuds (4) sur la membrane (3, 16).

12. Transducteur ultrasonore selon la revendication 1, dans lequel:
une surface d'un côté pour émettre et recevoir l'ultrason est revêtue avec un film de nano-revêtement (50).

13. Système de diagnostic endoscopique par ultrasons incluant le transducteur ultrasonore selon la revendication 1.

14. Système de diagnostic endoscopique par ultrasons selon la revendication 13, dans lequel:
le système de diagnostic endoscopique par ultrasons est un endoscope à ultrasons.

15. Système de diagnostic endoscopique par ultrasons selon la revendication 13, dans lequel:
le transducteur ultrasonore est adapté à être utilisé comme une sonde à ultrasons insérée dans un canal d'instrument dans un endoscope.

16. Système de diagnostic endoscopique par ultrasons selon la revendication 13, dans lequel:
le transducteur ultrasonore est adapté à être utilisé comme une sonde à ultrasons dans un vaisseau.

17. Système de diagnostic endoscopique par ultrasons selon la revendication 13, dans lequel:
le transducteur ultrasonore est adapté à être utilisé comme un endoscope à ultrasons de type capsule.

18. Procédé de production d'un transducteur ultrasonore micro-usiné incluant une pluralité de cellules (1, 25) de transducteur ultrasonore, chacune desquelles inclut un substrat (12) sur lequel une électrode inférieure (14, 80) est formée, et une membrane (3, 16) prévue à l'écart du substrat (12) et ayant une électrode supérieure (15) formée sur celle-ci, ledit transducteur ultrasonore générant un ultrason avec la membrane (3, 16) vibrant lorsqu'une tension est appliquée entre l'électrode inférieure (14, 80) et l'électrode supérieure (15), le procédé **caractérisé en ce que** comprenant:
la prévision d'un élément support (2, 13) sur le substrat pour supporter la membrane (3, 16) le long de noeuds (4) causés lorsque des vibrations fondamentalement libres sont causées dans la membrane, afin de supprimer une propagation de la vibration de chaque dite cellule (1, 25) de transducteur ultrasonore jusqu'à une cellule (1, 25) de transducteur ultrasonore adjacente ou jusqu'à un élément support (2, 13) de membrane.
